# EUROPÄISCHE PATENTSCHRIFT

(11) **EP 1 223 802 B1**
(45) Veröffentlichungstag und Bekanntmachung des Hinweises auf die Patenterteilung: **15.03.2006**
(21) Anmeldenummer: 00967562.0
(22) Anmeldetag: 12.09.2000
(51) Int. Cl.: A01K 67/027

(54) **ENTWICKLUNG EINER EFFEKTIVEN GENTRANSFERTECHNIK FÜR DAS EINSCHLEUSEN VON FREMD-DNS IN SPERMIENZELLEN**
DEVELOPMENT OF AN EFFECTIVE GENE TRANSFER TECHNIQUE FOR INTRODUCING FOREIGN DNA INTO SPERM CELLS
DEVELOPPEMENT D'UNE TECHNIQUE EFFICACE DE TRANSFERT GENETIQUE PERMETTANT D'INTRODUIRE UN DNS ETRANGER DANS DES CELLULES SPERMATIQUES

(30) Priorität: 16.09.1999 DE 19944402
(43) Veröffentlichungstag der Anmeldung: 24.07.2002
(73) Patentinhaber: Bios Biotechnologie Schönow GmbH, 16321 Schönow (DE)
(72) Erfinder: MÜLLER, Karin, 13125 Berlin (DE); MARKGRAF, Karin, 12459 Berlin (DE); SCHMIDT, Michael, F., G., 14554 Seddiner See (DE); HERRMAN, Andreas, 13156 Berlin (DE)
(74) Vertreter: Baumbach, Friedrich
(86) Internationale Anmeldenummer: PCT/DE2000/003164
(87) Internationale Veröffentlichungsnummer: WO 2001/019183

(56) Entgegenhaltungen:
- WO-A-95/01448
- FARRE, L. ET AL.: "Adenovirus-mediated introduction of DNA into pig sperm and offspring" MOLECULAR REPRODUCTION AND DEVELOPMENT, Bd. 53, Juni 1999 (1999-06), Seiten 149-158, XP000973900
- NUSSBAUM, O. UND LOYTER, A.: "Interaction of reconstituted Sendai viral envelopes with sperm cells: reconstituted Sendai virus envelope-induced fusion-mediated introduction of foreign material into bull sperm cells" ARCHIVES OF VIROLOGY, Bd. 140, Nr. 9, 1995, Seiten 1613-1622, XP000973621 in der Anmeldung erwähnt
- RAMANI, K. ET AL.: "Novel gene delivery to liver cells using engineered virosomes" FEBS LETTERS, Bd. 404, Nr. 2-3, 1997, Seiten 164-168, XP000942134
- SCHOEN, P. ET AL.: "Gene transfer mediated by fusion protein hemagglutinin reconstituted in cationic lipid vesicles" GENE THERAPY, Bd. 6, Nr. 5, Mai 1999 (1999-05), Seiten 823-832, XP000973629 in der Anmeldung erwähnt
- PONIMASKIN, E. ET AL.: "Sendai virosomes revisited: Reconstitution with exogenous lipids leads to potent vehicles for gene transfer" VIROLOGY, Bd. 269, 10. April 2000 (2000-04-10), Seiten 391-403, XP000941938

## Beschreibung

Die Erfindung betrifft die Entwicklung einer effektiven Gentransfertechnik für das Einschleusen von Fremd-DNS in Spermienzellen mittels virosomaler Carrier. Anwendungsgebiete der Erfindung sind die Landwirtschaft und die Biotechnologie.

Bezüglich der Transfektion von Spermien und deren Vektorfunktion für den Transport genetischen Materials in die Eizelle gibt es nur wenige Veröffentlichungen. Die Nutzung von Spermien als Überträger von Fremd-DNS in eine Eizelle wurde erstmalig von Lavitrano et al. (1989) bei der Maus beschrieben. 30% der erzeugten Nachkommen enthielten die Fremd-DNS. Spermien binden nach Lavitrano et al. (1992) die negativ geladene Plasmid-DNA über 30-35 kD Proteine nach einfacher Inkubation. Das von den Autoren 1989 beschriebene Verfahren konnte von anderen Gruppen nicht reproduziert werden (s. Bachiller et al., 1991). Letztere nutzten Liposomen-DNA-Komplexe (Lipofectin) zur Transfektion von Mäusespermien (80% Transfektionsrate). Bei normaler Befruchtungsrate konnten jedoch keine transgenen Nachkommen erzeugt werden.

Tsai et al. (1997) gelang nach Elektroporation von Spermien mariner Mollusken die Einschleusung fremder Plasmid-DNS und deren erfolgreiche Übertragung in die Eizelle bei einer Fertilisationsrate von >90%. 65% der Larven verfügten über die Fremd-DNS. Diese Elektroporation führt bei Säugerspermien zur Zerstörung des für den natürlichen Befruchtungsvorgang (also ohne intrazytoplasmatische Spermieninjektion ins Ei = ICSI) notwendigen Akrosoms (Nakanishi und Iritani, 1993).

Zur Virosomen-Spermien-Fusion gibt es 2 Arbeiten: Nussbaum et al. (1993) zeigten aus medizinischer Sicht (HIV usw.). daß Viren (Sendai, Influenza, Semliki forest) mit Bullenspermien über Rezeptormoleküle fusionieren können. Nussbaum und Loyter (1995) nutzten bereits rekonstruierte Virushüllen (Sendai), um den darin eingebauten Fluoreszensfarbstoff Calcein in Bullenspermien zu bringen. Bezüglich der Motilität der Spermien gab es jedoch nach der Fusion drastische Einschränkungen. Bei einem Virus-Partikel/Spermien-Verhältnis >10:1 sank die Motilität dramatisch. Darüber hinaus gibt es keine Angaben zum Prozentsatz fusionierter Spermien oder zum Targeting, da sich der benutzte photometrische Fusionsassay nur auf die Gesamtsuspension bezieht.

Die Nutzung von Virosomen oder Liposomen mit virosomalen Fusionsproteinen für einen gezielten Transfer verschiedenster Substanzen in Zellkulturen bzw. bestimmte Zielzellen in vivo (Targeting) wird von immer mehr Arbeitsgruppen angestrebt (US 5.683.866, Schoen et al., 1999). Bisher sind aber praktisch verwertbare Ergebnisse noch ausgeblieben.

In Archives of Virology 140(9), 1613-1622,1995 ist beschrieben, dass rekonstruierte Virushüllen von Sendai-Viren (RSVE) in der Lage sind, mit Bullenspermien zu fusionieren. RSVE mit darin eingebautem Fluoreszenzfarbstoff Calcein wurden mit Bullenspermien fusioniert, und anschließend konnte der Farbstoff in den Spermien nachgewiesen werden. Es wurde auch darauf hingewiesen, RSVE als Vehikel zu benutzen, um Fremd-DNA in Spermien einzuführen. Eine Einführung von Fremd-DNA wurde jedoch nicht realisiert. Bei der Rekonstruktion der Virosomen wurden außerdem keine exogenen Lipide zugesetzt.

Das Ziel der Erfindung besteht darin, transgene Spermien von Säugetieren (ausgenommen menschliche Spermien) zur Verfügung zu stellen, mit denen genetisches Material in die entsprechenden Eizellen eingeschleust werden kann.

Aufgabe der Erfindung ist, eine neue Gentransfertechnik zu entwickeln, die das Einschleusen von Fremd-DNS in Spermienzellen von Nutztieren, wie z.B. Bulle und Schafbock und deren nachfolgende Nutzung als Vektoren zur Erstellung transgener Embryonen gestattet. Das Verfahren soll die Eigenschaft viraler Bindungs- und Fusionsproteine, nämlich ein Verschmelzen von Lipidmembranen zu vermitteln, nutzen.

Das Ziel der Erfindung wird gemäß Anspruch 1 mit transgenen Spermien für Nutztiere erreicht, welche
- Fremd-DNA und
- rekonstituierte Virushüllen, die zusätzlich ein spezifisch definiertes exogenes Lipidgemisch beinhalten
enthalten.

Die Unteransprüche 2-7 sind Vorzugsvarianten.

Die Aufgabe der Erfindung der Herstellung solcher Spermien wird erfindungsgemäß gemäß den Ansprüchen 8-10 realisiert.

In einer ersten Phase wird die Herstellung von Virosomen und die Optimierung der Rekonstitutionsbedingungen hinsichtlich der Fusionsaktivität mit Spermienzellen standardisiert. Diese Arbeiten stellen den Schwerpunkt der Erfindung dar. Die detailliert dargelegten Versuche sind geeignete Ansätze bei der Realisierung (vgl.. Ergebnisse), wobei insbesondere die Reproduzierbarkeit der erarbeiteten Protokolle ein Hauptproblem darstellten, da die Vermehrung der benötigten Viren im Vorfeld der Virosomenherstellung offenbar zu relativ unterschiedlichen Populationen führt. Eine Angleichung der Präparationen wird erfindungsgemäß einerseits durch definierte und geeignete Zugabe exogener Lipide erreicht, andererseits wurden geeignete Präparationen durch die Optimierung der Lagerung gesplitteter Proben besser ausgenutzt.

Der Nachweis der Erfindung erfolgte unter Nutzung des Markergens für das sogenannte 'Grün Fluoreszierende Protein' (GFP), das sich als vorteilhaft für den Einbau eines Testgens in die Virosomen erwies. Die Transfektionseffizienz einiger Virosomenchargen konnte so parallel zu den Fusionsversuchen mit Spermienzellen auch anhand der Expression des GFP in einer CV-1-Zelllinie (Affennierenzellen) bewertet werden. Da Spermien keine Expression zeigen, müssen zum Nachweis der erfolgreichen Fusion mit Virosomen hier weitere Teste, z.B. ein Membran-Fusionstest auf Rhodamin-PE-Basis (vgl. Ausführungsbeispiel) genutzt werden.

Es wurden mehrere Virusstämme getestet, Sendai- und Influenza-Viren haben sich als bevorzugt erwiesen. Ein Influenzavirusstamm wurde bevorzugt ausgewählt. Ein bevorzugtes Lipidgemisch ist 3.5 µmol Phosphatidylethanolamin (PE), 36 µmol Phosphatidylcholin (PC), 62 µmol Sphingomyelin (SM), 62 µmol Cholesterol (C) für ein Viruskonzentrat mit einem Proteingehalt von ca. 25 - 30 mg. In Auswertung der Ergebnisse wurde der Schwerpunkt weiter auf die reproduzierbare Herstellung dieser Virosomen gelegt, indem möglichst einheitliche Viruspräparationen erzeugt und genutzt wurden.

Die Verwendung von Lipiden zur Herstellung von Virosomen zur Transfektion von Spermien wird mit der vorliegenden Erfindung erstmalig und mit Vorteil realisiert. Auch der Einsatz von Influenzaviren zum Aufbau von Virosomen gemäß der Erfindung ist absolut neu.

Parallel wurde die Spermienaufbereitung variiert, um die beste Überlebensrate bzw. später Befruchtungsrate nach Fusion zu erreichen.

### Ergebnisse

Für die Virosomenherstellung wurden zwei verschiedene Virusstämme getestet: Sendai-Viren und Influenza-Viren. Beide Viren und damit auch die daraus gewonnenen Virosomen haben den Vorteil, daß sie mit vielen Zellarten unterschiedlicher Spezies fusionieren, einschließlich Bullenspermienzellen.

Zur Herstellung der Virosomen wurde die Virushülle von dem viralen Erbgut abgetrennt. Die aufgelösten Virushüllbestandteile enthalten sowohl Lipide als auch die für die Fusion wichtigen Proteine. Unter Zusatz verschiedener exogener Lipide wurden Virosomen konstruiert, die entweder Rhodamin-PE als Membranmarker oder das eingebaute GFP-Plasmid enthalten, das ein grünes Fluoreszenzprotein kodiert.
Als Kontrolle, ob das GFP exprimiert wird, werden die Virosomen auch auf CV-1 Zellen (Affennierenzellen) getestet. Für die spätere anwendungsreife Nutzung des virosomalen Carriers sollen andere Genkonstrukte verwendet werden.

**Tabelle 1: Darstellung der Ergebnisse, die mit verschiedenen Varianten von Virosomen erreicht wurden.**

| | | | | | | |
|---|---|---|---|---|---|---|
| **Viren für Virosomenherstellung** | Sendai | Sendai | Influenza | Influenza | Influenza | Influenza |
| **Lipidzusatz für die Virosomenherstellung** | Cholesterol, Sphingomyelin, Phosphatidy Icholin, Phosphatidy lethanolamin | ohne | Cholesterol, Sphingomyelin, Phosphatidy Icholin, Phosphatidy lethanolamin | DOTAP, Phosphati dylethanolamin | Phosphati dylethanolamin | ohne |
| **Fusionsrate** | 90-95% | 5 - 10 % | 90-95% | 90% | 85-90% | 65 % |
| **Motilität** | < 5 % | - | 90 % | 50 - 60 % | 90 % | 85 % |
| **Fusionsregion an den Spermienzellen** | am gesamSpermium ten | - | am gesamten Spermium | gesamter Spermienschwanz, nicht am Kopf | Kopf, Mittelstück -des Schwanze s | unterschiedlich |

| | | | | | | |
|---|---|---|---|---|---|---|
| Sendai-Virosomen: Virosomen aus Sendai Viren zeigten eine hohe Fusionsrate mit den Spermien. Die Fusion kann bei pH 7 durchgeführt werden, was für die Zellen vorteilhaft ist, da keine unphysiologische pH-Wert-Erniedrigung nötig ist. Rund 90 - 95 % der Spermien, die mit Rhodamin-PE-markierten Virosomen behandelt wurden, zeigten danach ebenfalls diese Markierung als Zeichen für die Membranverschmelzung. Die Beweglichkeit und die Lebensfähigkeit dieser Spermien jedoch wurden durch die Sendai-Virosomen stark eingeschränkt. Weniger als 5 % der Spermien zeigten nach der Fusion mit den Sendai-Virosomen noch gute Motilität und Vitalität.Der Großteil der fusionierten Spermien war eingeschränkt bzw. nicht beweglich. Teilweise wurde durch die Sendai-Virosomen das Akrosom der Spermien beschädigt. Ein intaktes Akrosom, gute Beweglichkeit und das mit großer Spermienanzahl sind aber Vorraussetzungen für eine erfolgreiche Befruchtung. Auch andere Varianten (Eliminierung von Virusprotein) und kürzere Inkubationszeiten veränderten nicht die Toxizität dieser Virosomen. | | | | | | |

**Tabelle 2: Darstellung der Ergebnisse, die mit verschiedenen Varianten von Virosomen erreicht wurden (jeweils 3 bzw. 4 verschiedene Präparationen pro Variante)**

| | | |
|---|---|---|
| **Viren für Virosomen herstellung** | Influenza | Influenza |
| **Lipidzusatz für die Virosomenherstellung** | Cholesterol, Sphingomyelin, Phosphatidylcholin, Phosphatidylethanolamin | Phosphatidylethanolamin (PE), SAINT-PE |
| **Fusionsrate [%]** | 67/96/94/91 | 84/93/92 |
| **Vitalität [% vitale]** | 100/100/98/93 | 94/100/100 |
| **% vitale nicht** | 13/0/0/0 | 1/0/4 |
| **fusionierte Spermien** | | |

Das bereits benannte Lipidgemisch wurde als Zusatz bei der Virosomenherstellung im Vergleich zu einem Gemisch aus jeweils 100 µmol Phosphatidylethanolamin (PE) und 100 µmol SAINT-PE für ein Viruskonzentrat mit einem Proteingehalt von ca. 25 - 30 mg genutzt. Die Fusion der Spermienzellen mit den Virosomen wurde nach Selektion der motilen Zellen durch eine geeignete schonende Verschiebung des pH-Wertes auf 5 und anschließende Reneutralisierung erreicht. Die Vitalität der Spermien wurde nach der Fusion mittels Rhodamin 123 getestet, einem Fluoreszenzmarker für mitochondriale Aktivität. Parallel erfolgte die Darstellung der Fusion mit den Virosomen mit Hilfe des in die Virosomenmembran eingebauten Rhodamin-PE (wie bereits beschrieben).

Beide Lipidzusätze erbrachten eine reproduzierbar hohe Fusionsrate unter Erhalt der Spermienvitalität. Bei Nutzung der Lipidmischung (Cholesterol, Sphingomyelin, Phosphatidylcholin, Phosphatidylethanolamin) wurde mikroskopisch mit drei von vier Virosomenpräparationen kein vitales Spermium beobachtet, das nicht auch fusioniert war. Damit ist mit hoher Wahrscheinlichkeit ausgeschlossen, daß nicht fusionierte/transgene Spermien zur Befruchtung gelangen.

Durch die Erfindung wurden grundlegende Resultate für die Nutzung eines virosomalen Carriers geschaffen.

Die Vorteile der neuen Methode liegen vor allem darin:
- Eine Transfizierung fast aller lebenden Spermien einer Probe wird erreicht
- Die Befruchtungsfähigkeit wird ohne zusätzliche Manipulation erhalten
- Ein Targeting an bestimmte Spermienregionen ist möglich
- Es können genügend große DNA-Mengen (Virosomengröße oder Anzaht/Spermium) eingebracht werden.

Die Erfindung wird nachfolgend durch ein weiteres Ausführungsbeispiel näher erläutert.

### Influenza-Virosomen:

Influenza-Virus und die daraus gewonnenen Virosomen wurden bei pH 7 an Bullenspermien gebunden. Erst eine kurzzeitige pH-Erniedrigung auf pH 5 löste dann die Fusion aus. Anschließend wurde der pH der Spermiensuspension wieder auf pH 7 reneutralisiert, damit die Spermienzellen durch die pH-Änderung nicht unnötigem Stress ausgesetzt werden. Die Fusionsrate lag zwischen 60 - 90 % in Abhängigkeit von den zugesetzten Lipiden. Es wurden Virosomen aus zwei unterschiedlichen Influenza-Stämmen (APR 8, X-31) hergestellt. Ein Einfluß auf die Fusionsrate konnte nicht festgestellt werden. Die Toxizität der Influenza-Virosomen war deutlich geringer als die der Sendai-Virosomen. Je nach Lipidzusammensetzung der Virosomen zeigten die Bullenspermien Beweglichkeiten von 50 - 90 %. Die höchste Fusionsrate mit minimaler Toxizität erreichten wir mit Influenza-Virosomen, denen ein Lipidgemisch aus vier verschiedenen Lipiden (Cholesterol, Sphingomyelin, Phosphatidaylcholin, Phosphatidylethanolamin) zugesetzt wurde. Bei den Virosomen mit diesem Lipidgemisch zeigte sich auch, daß die Fusion am gesamten Spermium stattfindet. Influenza-Virosomen, die mit anderen Lipiden hergestellt wurden, wiesen unterschiedlich bevorzugte Fusionsregionen an den Spermienzellen auf. Für das Einschleusen in die Rindereizelle durch Befruchtung ist es Vorraussetzung, daß das Genkonstrukt im Kopf oder Hauptstück des Schwanzes der Bullenspermienzellen lokalisiert ist. Influenza-Virosomen, die mit diesem Lipidgemisch (Cholesterol, Sphingomyelin, Phosphatidaylcholin, Phosphatidylethanolamin) hergestellt wurden, erfüllen die Vorraussetzungen, die eine weitere Befruchtung möglich machen.

Nachweis der Fusion mit Spermien erfolgt mikroskopisch über:
- Rhodamin-Phosphatidylethanolamin (Rh-PE) in der Virosomenmembran, eingebaut bei der Rekonstruktion: Gebundene Virosomen sind im Fluoreszenzmikroskop als rote Spots sichtbar. Nach Fusion zeigen fusionierte Membranareale infolge des Fluoreszenzdequenching (üblicher Fusionsassay) eine leuchtend rote kontinuierliche Floureszenz.
- Der Fluoreszenzfarbstoff Hoechst 33342 (H 342), gebunden an das GFP-Plasmid vor der Rekonstruktion der Virosomen: Überschüssiger Farbstoff wird durch Waschung von den Virosomen entfernt. Der fluoreszierende DNS-Farbstoff ist in der Lage Zellmembranen zu permeieren. Bei Vorhandensein von überschüssigem Farbstoff wären daher alle Zellen gefärbt. Bei Färbung einzelner Zellen muß davon ausgegangen werden, daß h 342 über die Virosomenfusion in die Zelle gelangt ist.

Im Vergleich zur radioaktiven Markierung der Fremd-DNS gestatten die fluoreszenzoptischen Assays die Einzelzellanalyse.

### Literatur:

Schoen, P. et al. (1999) Gene Therapy 6, 823 832
Tsai, H.-J.et al. (1997) Transgenic Res. 6, 85-95
Bachiller, D. et al. (1991) Molec. Reprod. Dev. 30, 194-200
Lavitrano, M. et al. (1989) Cell 57, 717-723
Lavitrano, M. et al. (1992) Molec. Reprod. Dev. 31, 161-169
Nakaanishi, A. et al. (1993) Molec. Reprod. And Dev. 36, 258-261
Nussbaum, O. et al. (1993) Exp. Cell Res. 206, 11-15
Nussbaum, O. et al. (1995) Arch. of Virology 140, 1613-1622

## Patentansprüche

1. Transgene Spermien, umfassend Säugetierspermien, ausgenommen menschliche Spermien, von Nutztieren, Fremdgenkonstrukte in Form von Virosomen aus Bestandteilen der Hülle eines Virus sowie eines definierten exogenen Lipidgemisches aus mindestens einem amphiphilen Lipid und gegebenenfalls einem Steroid als virosomale Carrier, **dadurch gekennzeichnet, dass** sie
- rekonstituierte Virushüllen von Sendai-Viren oder von Influenza-Viren enthalten,
- die zusätzlich ein spezifisches exogenes Lipidgemisch aus Cholesterol, Sphingomyelin, Phosphatidylcholin und Phosphatidylethanolamin bzw. aus Phosphatidylethanolamin und DOTAP oder SAINT-PE bzw. aus Phosphatidylethanolamin beinhalten.

2. Transgene Spermien nach Anspruch 1, **dadurch gekennzeichnet, dass** die Virosomen aus Lipid- und/oder Proteinbestandteilen der Hülle von Sendai-Viren und einem exogenen Lipidgemisch aus Cholesterol, Sphingomyelin, Phosphatidylcholin und Phosphatidylethanolamin bestehen.

3. Transgene Spermien nach Anspruch 1, **dadurch gekennzeichnet, dass** die Virosomen aus Lipid- und/oder Proteinbestandteilen der Hülle von Influenza-Viren und einem exogenen Lipidgemisch aus Cholesterol, Sphingomyelin, Phosphatidylcholin und Phosphatidylethanolamin bestehen.

4. Transgene Spermien nach Anspruch 1, **dadurch gekennzeichnet, dass** die Virosomen aus Lipid- und/oder Proteinbestandteilen der Hülle von Influenza-Viren und einem exogenen Lipidgemisch aus Phosphatidylethanolamin oder aus Phosphatidylethanolamin und DOTAP oder aus Phosphatidylethanolamin und SAINT-PE bestehen.

5. Transgene Spermien nach Anspruch 1, **dadurch gekennzeichnet, dass** die Virosomen aus Lipid- und/oder Proteinbestandteilen der Hülle von Influenza-Viren und einem exogenen Lipidgemisch aus Phosphatidylethanolamin bestehen.

6. Transgene Spermien nach einem der Ansprüche 1 bis 5, **dadurch gekennzeichnet, dass** die Fremdgenkonstrukte Nachweisgenkonstrukte, vorzugsweise für GFP oder Luciferase sind.

7. Transgene Spermien nach einem der Ansprüche 1 bis 5, **dadurch gekennzeichnet, dass** die Fremdgenkonstrukte Gene für Proteine sind, die pharmazeutisch benutzt werden.

8. Verfahren zur Herstellung von befruchtungsfähigen transgenen Spermien nach Anspruch 1-7, **dadurch gekennzeichnet, dass** mit Fremdgenkonstrukten Virosome hergestellt und mit Säugetierspermien, ausgenommen menschliche Spermien, von Nutztieren, fusioniert werden.

9. Transgene Spermien nach Anspruch 1, **dadurch gekennzeichnet, dass** sie als transgene Spermien von Rindern oder Schafen gebildet sind.

10. Verfahren nach Anspruch 8, **dadurch gekennzeichnet, dass** die Virosome mit Spermien von Rindern oder Schafen fusioniert werden.

## Claims

1. Transgenic sperms, entailing mammal sperms, with the exception of human sperms, of domestic animals, outside gene constructs in the form of virosomes from components of the coat of a virus as well as a defined exogenous lipid mixture of at least one amphiphile lipid and, if applicable, a steroid as a virosomal carrier, wherein they
- contain reconstructed virus coats of Sendai viruses or of influenza viruses,
- they additionally contain a specific exogenous lipid mixture of cholesterol, sphingomyelin, phosphatidylcholine and phosphatidylethanolamine or of phosphatidylethanolamine and DOTAP or SAINT-PE or of phosphatidylethanolamine, as the case may be.

2. Transgenic sperms according to Claim 1, wherein the virosomes comprise lipid and/or protein components of the coat of Sendai viruses and an exogenous lipid mixture of cholesterol, sphingomyelin, phosphatidylcholine and phosphatidylethanolamine.

3. Transgenic sperms according to Claim 1, wherein the virosomes comprise lipid and/or protein components of the coat of influenza viruses and an exogenous lipid mixture of cholesterol, sphingomyelin, phosphatidylcholine and phosphatidylethanolamine.

4. Transgenic sperms according to Claim 1, wherein the virosomes comprise lipid and/or protein components of the coat of influenza viruses and an exogenous lipid mixture of phosphatidylethanolamine or of phosphatidylethanolamine and DOTAP or of phosphatidylethanolamine and SAINT-PE.

5. Transgenic sperms according to Claim 1, wherein the virosomes comprise lipid and/or protein components of the coat of influenza viruses and an exogenous lipid mixture of phosphatidylethanolamine.

6. Transgenic sperms according to one of the Claims 1 to 5, wherein the outside gene constructs are detection gene constructs, preferably for GFP or luciferase.

7. Transgenic sperms according to one of the Claims 1 to 5, wherein the outside gene constructs are genes for proteins which are used pharmaceutically.

8. Method for the production of fertilisation-capable transgenic sperms according to one of the Claims 1 to 7, wherein virosomes are produced with outside gene constructs and are fused with mammal sperms, with the exception of human sperms, from domestic animals.

9. Transgenic sperms according to Claim 1, wherein they are formed as transgenic sperms of cattle or sheep.

10. Method according to Claim 8, wherein the virosomes are fused with sperms of cattle or sheep.

## Revendications

1. Spermatozoïdes transgéniques, englobant les spermatozoïdes de mammifères, à l'exception des spermatozoïdes humains, d'animaux de rapport, de constructions géniques externes sous forme de virosomes composés d'éléments de l'enveloppe d'un virus ainsi que d'un mélange lipidique exogène défini composé d'au moins un lipide amphiphile et le cas échéant d'un stéroïde en tant que transporteur virosomal, **se caractérisant par le fait qu'**ils
- contiennent des enveloppes reconstituées de virus Sendai ou de virus influenza,
- qui contiennent en plus un mélange lipidique exogène spécifique composé de cholestérol, de sphingomyéline, de phosphatidylcholine et de phosphatidyléthanolamine voire de phosphatidyléthanolamine et de DOTAP ou de SAINT-PE voire de phosphatidyléthanolamine.

2. Spermatozoïdes transgéniques selon la revendication 1, **se caractérisant par le fait que** les virosomes se composent d'éléments lipidiques et/ou protéiniques de l'enveloppe de virus Sendai et d'un mélange lipidique exogène composé de cholestérol, sphingomyéline, phosphatidylcholine et phosphatidyléthanolamine.

3. Spermatozoïdes transgéniques selon la revendication 1, **se caractérisant par le fait que** les virosomes se composent d'éléments lipidiques et/ou protéiniques de l'enveloppe de virus influenza et d'un mélange lipidique exogène composé de cholestérol, sphingomyéline, phosphatidylcholine et phosphatidyléthanolamine.

4. Spermatozoïdes transgéniques selon la revendication 1, **se caractérisant par le fait que** les virosomes se composent d'éléments lipidiques et/ou protéiniques de l'enveloppe de virus influenza et d'un mélange lipidique exogène composé de phosphatidyléthanolamine ou de phosphatidyléthanolamine et DOTAP ou de phosphatidyléthanolamine et SAINT-PE.

5. Spermatozoïdes transgéniques selon la revendication 1, **se caractérisant par le fait que** les virosomes se composent d'éléments lipidiques et/ou protéiniques de l'enveloppe de virus influenza et d'un mélange lipidique exogène composé de phosphatidyléthanolamine.

6. Spermatozoïdes transgéniques selon les revendications 1 à 5, **se caractérisant par le fait que** les constructions géniques externes sont des constructions géniques codant d de préférence le GFP ou la luciférase.

7. Spermatozoïdes transgéniques selon l'une des revendications 1 à 5, **se caractérisant par le fait que** les constructions géniques externes sont des gènes pour des protéines qui sont utilisées en pharmacie.

8. Procédé de fabrication de spermatozoïdes transgéniques inséminables selon l'une des revendications 1 à 7, **se caractérisant par le fait que** les virosomes sont fabriqués avec es constructions géniques externes et sont fusionnés avec des spermatozoïdes de mammifères, à l'exception de spermatozoïdes humains, d'animaux de rapport.

9. Spermatozoïdes transgéniques selon la revendication 1, **se caractérisant par le fait qu'**ils se forment de spermatozoïdes transgéniques de boeufs ou de moutons.

10. Procédé selon la revendication 8, **se caractérisant par le fait que** les virosomes sont fusionnés avec des spermatozoïdes de boeufs ou de moutons.
